# EUROPEAN PATENT APPLICATION

(11) **EP 4 335 483 A1**
(43) Date of publication of application: **13.03.2024**
(21) Application number: 23196489.1
(22) Date of filing: 11.09.2023
(51) Int. Cl.: A61M 37/00

(54) **TATTOO NEEDLE CARTRIDGE**

(30) Priority: 09.09.2022 US 202263405180 P
(71) Applicant: 2350445 Ontario Limited, Toronto, ON M6P 1Z2 (CA)
(72) Inventor: CHEN, Tina Ming, Toronto, Ontario, M4R 1R8 (CA)
(74) Representative: ABG Intellectual Property Law, S.L.

(57) **Abstract**

A tattoo needle cartridge (100) comprising: a body portion (104) housing a needle assembly (124); and an outlet portion (112) on a first end of the body portion, the outlet portion including: a barrel (308) with: a first barrel end (312) adjacent to the body portion; a second barrel end (316) opposite the first barrel end forming an outlet of the cartridge; and a barrel body (320) having: an inner bore (324) extending from the first barrel end to the second barrel end; and a forked barrel tip (336) with two prongs (340,340a,340b) extending from the barrel body towards the second barrel end.

## Description

### FIELD

The specification relates generally to tattooing devices, and specifically to tattoo needle cartridges.

### BACKGROUND

Tattoo artists draw designs on skin by pressing reciprocating tattoo needles on the skin. The tattoo needles are contained in tattoo needle cartridges. As the needles advance into the skin the needles open up holes on the skin onto which ink is drawn when the needles retract from the skin. The ink is delivered through an opening of the cartridges. Ink may accumulate in the opening periodically discharging a droplet of ink, which can occlude a working area, leading to time spent cleaning the working area, and ink waste.

### SUMMARY

An aspect of the specification provides a tattoo needle cartridge comprising: a body portion housing a needle assembly; and an outlet portion on a first end of the body portion, the outlet portion including: a barrel with: a first barrel end adjacent to the body portion; a second barrel end opposite the first barrel end forming an outlet of the cartridge; and a barrel body having: an inner bore extending from the first barrel end to the second barrel end; and a forked barrel tip with two prongs extending from the barrel body towards the second barrel end.

Another aspect of the specification provides an outlet portion for a tattoo needle cartridge comprising: a barrel with: a first barrel end for attachment to the cartridge; a second barrel end opposite the first barrel end forming an outlet of the cartridge; and a barrel body having: an inner bore extending from the first barrel end to the second barrel end; and a forked barrel tip with two prongs extending from the barrel body towards the second barrel end.

### BRIEF DESCRIPTIONS OF THE DRAWINGS

The accompanying figures, where like reference numerals refer to identical or functionally similar elements throughout the separate views, together with the detailed description below, are incorporated in and form part of the specification, and serve to further illustrate embodiments of concepts that include the claimed invention and explain various principles and advantages of those embodiments.
FIG. 1A depicts a front view of an example tattoo needle cartridge.
FIG 1B depicts a cross-section view of the example tattoo needle cartridge of FIG. 1A.
FIG. 2 depicts a perspective view of a cartridge body of the tattoo needle cartridge depicted in FIG. 1A.
FIG. 3A depicts a perspective view of a prior-art outlet portion for a cartridge body with a needle.
FIG. 3B depicts an enlarged view of the outlet portion of the cartridge body depicted in FIG. 2.
FIG. 4 depicts another perspective view of the outlet portion of the cartridge body depicted in FIG. 2.
FIG. 5A depicts another perspective view of the alternative outlet portion of FIG. 3A showing a meniscus of ink at an opening of the outlet portion.
FIG 5B depicts another perspective view of the outlet portion of FIG. 3B showing a meniscus of ink traveling towards an end of the outlet portion.
FIG. 6A depicts a cross-section view of another example tattoo needle cartridge.
FIG. 6B depicts a cross-section view of another example tattoo needle cartridge.

Skilled artisans will appreciate that elements in the figures are illustrated for simplicity and clarity and have not necessarily been drawn to scale. For example, the dimensions of some of the elements in the figures may be exaggerated relative to other elements to help to improve understanding of embodiments of the present invention.

The components have been represented where appropriate by conventional symbols in the drawings, showing only those specific details that are pertinent to understanding the embodiments of the present invention so as not to obscure the disclosure with details that will be readily apparent to those of ordinary skill in the art having the benefit of the description herein.

### DETAILED DESCRIPTION

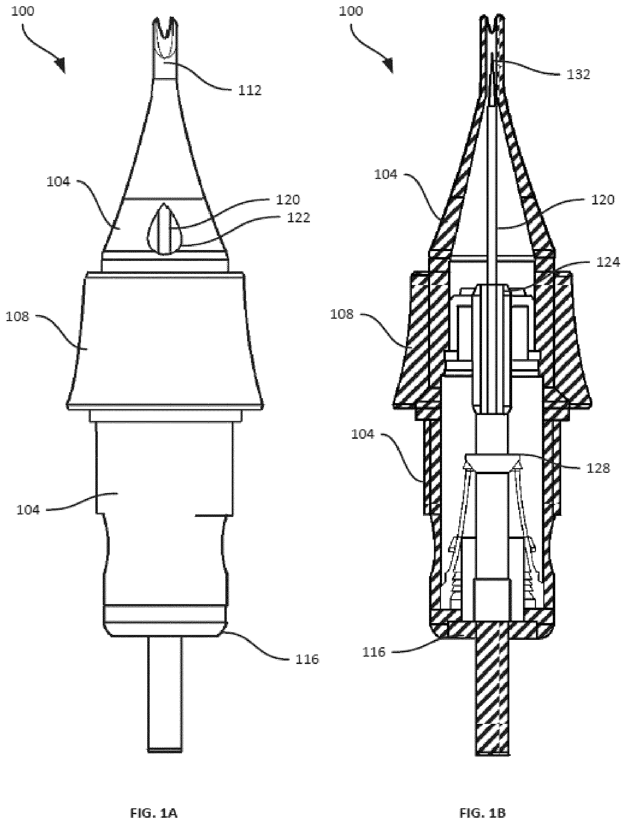

FIG. 1A depicts a tattoo needle cartridge 100, including a body portion 104 with a grip portion 108, an outlet portion 112 on a first end of the body portion 104, and a base portion 116 on a second end of the body portion 104 opposite the outlet portion 112. The body portion 104 surrounds the base portion 116 and houses a needle 120 that is partially visible in FIG. 1A through an opening 122 in the body portion 104. The outlet portion 112 may be detachable from the body portion 104.

FIG 1B. depicts a cross-section view of the body 104 of the tattoo needle cartridge 100 depicting the needle 120 mounted on a needle assembly 124. The needle assembly 124 is supported inside the body portion 104 by a needle holder 128. The needle holder 128 may be supported by the base portion 116. The needle holder 128 and the needle assembly 124 may reciprocate within the body portion 104, e.g., when the needle assembly 124 is actuated by an apparatus (not shown) on which the cartridge 100 is mounted and from which ink can be drawn from a reservoir into the cartridge 100. The ink travels to a tip 132 of the needle 116, which extends from the outlet portion 112, for application to skin or other suitable substrate.

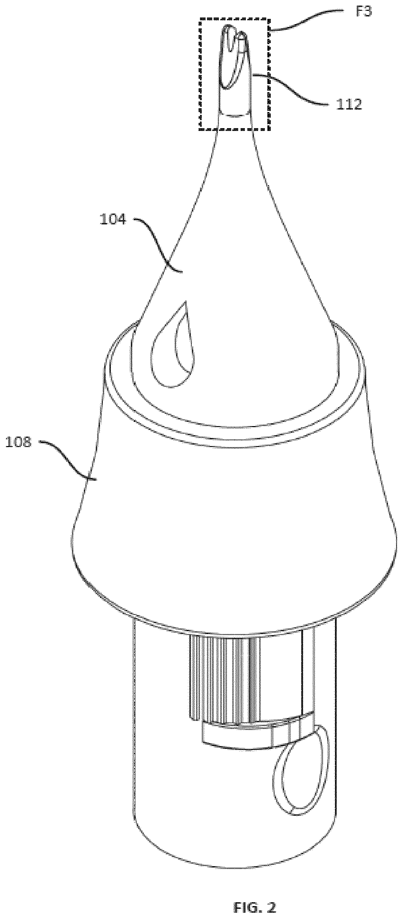

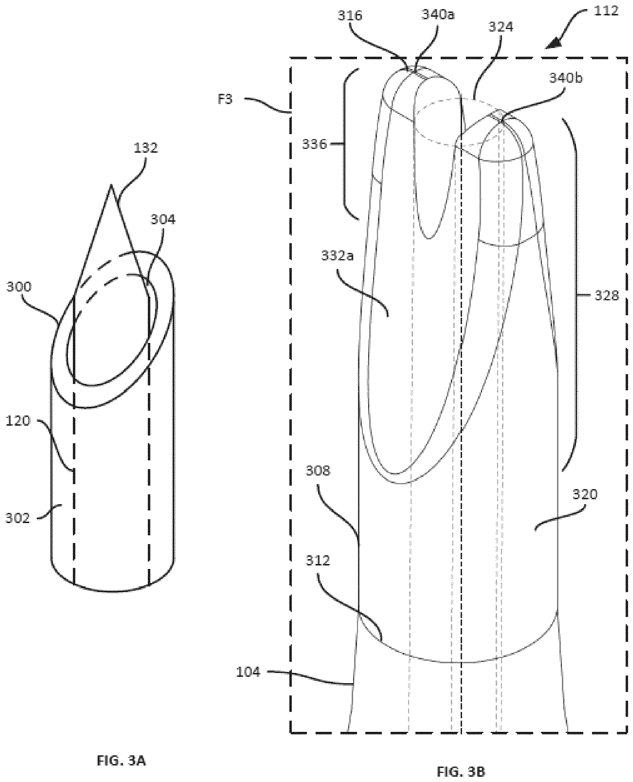

FIG. 2 shows a simplified view of the body portion 104, without the needle assembly 124 and without the holder 128. The outlet portion 112, bounded in a dashed box F3, is shown in greater detail in FIG. 3B.

FIG. 3A illustrates a prior-art outlet portion 300, with a cylindrical barrel 302 terminating in a flat circular opening (e.g., perpendicular to the barrel 302) or an angled elliptical opening 304 (as illustrated). FIG. 3A further illustrates the needle 120 extending through the barrel 302 with the needle tip 132 protruding from the opening 304. The outlet portion 300 is prone to an accumulation of ink at the opening 304 and may therefore periodically discharge a droplet of ink larger than desired, e.g., requiring cleaning of a work surface on which the ink is being applied.

The outlet portion 112 shown in detail in FIG. 3B mitigates such accumulation of ink. In particular, the outlet portion 112 includes a barrel 308 with a first barrel end 312 adjacent a first end of the body portion 104. The barrel 308 also has a second barrel end 316 opposite the first barrel end 312 forming an outlet of the cartridge 100. The barrel also has a barrel body 320 with an inner bore 324 extending from the first barrel end 312 to the second barrel end 316 defining a channel in communication with an interior of the body portion 104 for the ink to exit the cartridge 100. The needle tip 120 may reciprocate in the inner bore 324 and the needle tip 132 may protrude from the second barrel end 316 when the needle assembly 124 is actuated. The barrel body 320 further includes a tapered portion 328 formed by two cutouts 332 (one cutout 332a is visible in FIG. 3B) extending through the barrel body 320 from an outer surface of the barrel body 320 to the inner bore 324. The tapered portion 328 defines a forked barrel tip 336 with two prongs 340 (340a and 340b) extending towards the second barrel end 316.

Alternatively, instead of the tapered portion 328 defining the forked barrel tip 336, the tapered potion 328 may be omitted and the forked barrel tip 336 may be defined, for example, by protrusions attached to the second barrel body 320. Alternatively, the tapered portion 328 may be formed by more than two cutouts 332, for example, three cutouts 332, defining a forked barrel tip 336 having three prongs 340. The prongs 340 reduce an area of contact between the ink and the outlet portion 112 as the ink travels towards the second barrel end 316.

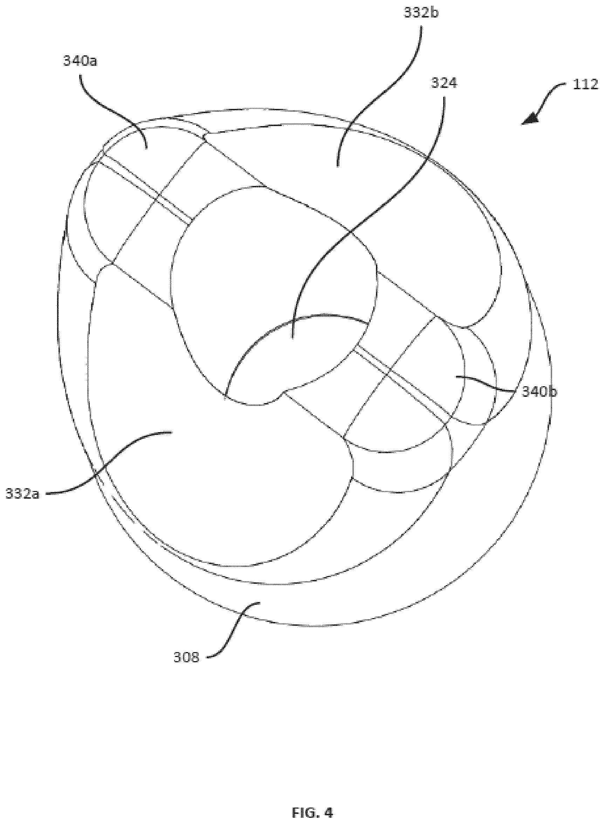

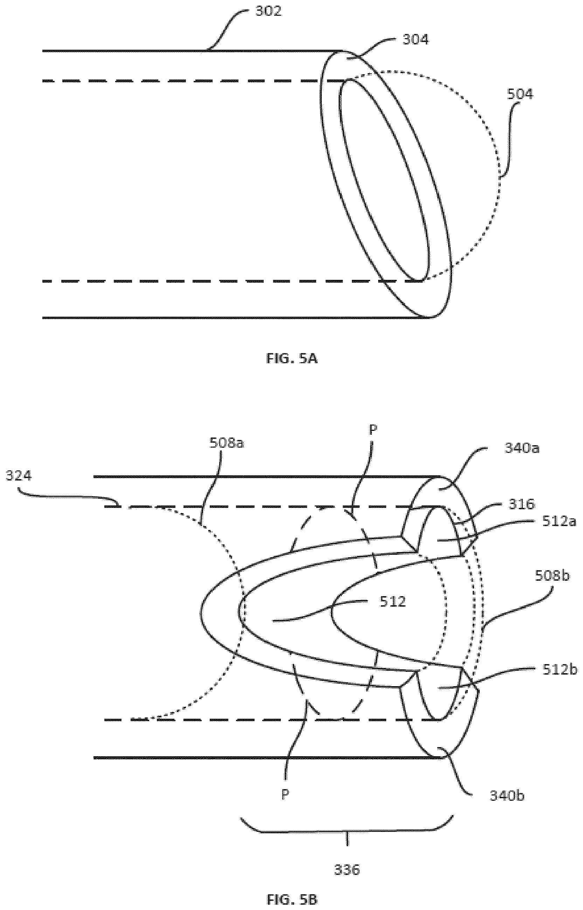

FIG. 4 illustrates the outlet portion 112 from above, rendering the inner bore 324, the two cutouts 332a and 332b, and the two prongs 340a and 340b more readily visible. The forked barrel tip 336 mitigates against ink accumulation as it is further explained below with reference to FIG. 5A and 5B.

FIG. 5A illustrates ink flowing through the prior-art outlet portion 300. A substantially half-spherical meniscus 504 of ink is shown forming at the opening 304, resulting in a large droplet of ink.

FIG. 5B illustrates ink flowing through the outlet portion 112. FIG. 5B shows the ink with as it travels through the inner bore 324 towards the second barrel end 316 due to capillary action between the ink and a wall 512 of the inner bore 324. Before reaching the forked barrel tip 336, a substantially half-spherical meniscus 508a (of substantially the same shape of the meniscus 504) is formed. As the ink moves through the forked barrel tip 336, the shape of the meniscus 508 starts deforming to conform to a shape defined by a cross-sectional perimeter P of the wall 512. The perimeter P becomes gradually smaller towards the second barrel end 316, as defined by inner prong surfaces 512a and 512b (which are a continuation of the wall 512 at the forked barrel tip 336). As a result, a meniscus 508b of a lower surface area to the meniscus 508a is formed at the second barrel end 316, resulting in smaller droplets of ink.

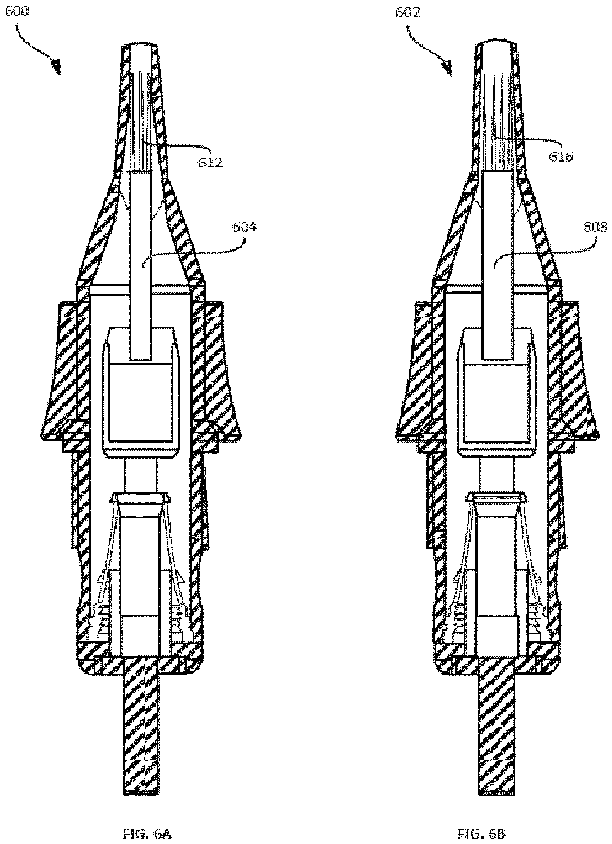

The needle assembly 124 may contain more than one needle 120. FIGS. 6A and 6B depict further example tattoo needle cartridges 600 and 602 comprising needle assemblies 604 and 608, each containing a bundle of three needles 612 and a bundle of four needles 616, respectively.

The benefits, advantages, solutions to problems, and any element(s) that may cause any benefit, advantage, or solution to occur or become more pronounced are not to be construed as a critical, required, or essential features or elements of any or all the claims. The invention is defined solely by the appended claims including any amendments made during the pendency of this application and all equivalents of those claims as issued.

The scope of the claims should not be limited by the embodiments set forth in the above examples but should be given the broadest interpretation consistent with the description as a whole.

## Claims

1. A tattoo needle cartridge comprising:
a body portion housing a needle assembly; and
an outlet portion on a first end of the body portion, the outlet portion including:
a barrel with:
a first barrel end adjacent to the body portion;
a second barrel end opposite the first barrel end forming an outlet of the cartridge; and
a barrel body having:
an inner bore extending from the first barrel end to the second barrel end; and
a forked barrel tip with two prongs extending from the barrel body towards the second barrel end.

2. The tattoo needle cartridge of claim 1 wherein the forked barrel tip defines two inner prong surfaces of the two prongs with a cross-sectional perimeter decreasing towards the second barrel end.

3. The tattoo needle cartridge of claim 1 wherein the barrel body further comprises a tapered portion formed by two cutouts extending through the barrel body from an outer surface of the barrel body to the inner bore, the tapered portion defining the forked barrel tip.

4. The tattoo needle cartridge of claim 1 further comprising:
a base portion on a second end of the body portion opposite the outlet portion including a needle holder;
wherein the needle holder supports the needle assembly, the needle assembly supporting a needle.

5. The tattoo needle cartridge of claim 4 wherein the needle assembly supports at least two needles.

6. The tattoo needle cartridge of claim 4 wherein the needle holder and the needle assembly reciprocate within the body portion when the needle assembly is actuated.

7. The tattoo needle cartridge of claim 6 wherein a needle tip of the needle protrudes from the forked barrel tip when the needle assembly is actuated.

8. The tattoo needle cartridge of claim 1 wherein the outlet portion is detachable from the body portion.

9. The tattoo needle cartridge of claim 1 wherein the forked barrel tip comprises at least three prongs extending from the barrel body towards the second barrel end.

10. An outlet portion for a tattoo needle cartridge comprising:
a barrel with
a first barrel end for attachment to the cartridge;
a second barrel end opposite the first barrel end forming an outlet of the cartridge; and
a barrel body having:
an inner bore extending from the first barrel end to the second barrel end; and
a forked barrel tip with two prongs extending from the barrel body towards the second barrel end.

11. The outlet portion of claim 10 wherein the forked barrel tip defines two inner prong surfaces of the two prongs with a cross-sectional perimeter decreasing towards the second barrel end.

12. The outlet portion of claim 10 wherein the barrel body further comprises a tapered portion formed by two cutouts extending through the barrel body from an outer surface of the barrel body to the inner bore, the tapered portion defining the forked barrel tip.

13. The outlet portion of claim 10 wherein the forked barrel tip comprises at least three prongs extending from the barrel body towards the second barrel end.
